(19) Europäisches Patentamt
European Patent Office
Office européen des brevets

(11) EP 1 001 740 B1

(12) **FASCICULE DE BREVET EUROPEEN**

(45) Date de publication et mention de la délivrance du brevet:
**15.10.2003 Bulletin 2003/42**

(21) Numéro de dépôt: **98941569.0**

(22) Date de dépôt: **07.08.1998**

(51) Int Cl.7: **A61K 7/48**

(86) Numéro de dépôt international:
**PCT/FR98/01770**

(87) Numéro de publication internationale:
**WO 99/007338 (18.02.1999 Gazette 1999/07)**

(54) **UTILISATION DU GINSENOSIDE RB1 POUR STIMULER LA SYNTHESE DE L'ELASTINE**

VERWENDUNG VON GINSENOSIDE RB1 ZUR STIMULIERUNG DER ELASTINSYNTHESE

USE OF THE Rb 1 GINSENOSIDE FOR STIMULATING ELASTIN SYNTHESIS

(84) Etats contractants désignés:
**DE ES FR GB IT**

(30) Priorité: **08.08.1997 FR 9710204**

(43) Date de publication de la demande:
**24.05.2000 Bulletin 2000/21**

(73) Titulaire: **LVMH RECHERCHE**
**75008 Paris (FR)**

(72) Inventeurs:
- **MEYBECK, Alain**
  **F-92400 Courbevoie (FR)**
- **DUMAS, Marc**
  **F-45100 Orléans (FR)**
- **GONDRAN, Catherine**
  **F-45730 SAINT BENOIT SUR LOIRE (FR)**
- **BONTE, Frédéric**
  **F-45100 Orléans (FR)**

(74) Mandataire: **Giraud, Françoise et al**
**Cabinet Beau de Loménie**
**158, rue de l'Université**
**75340 Paris Cedex 07 (FR)**

(56) Documents cités:
**EP-A- 0 283 713** **WO-A-97/06659**
**FR-A- 2 695 561**

- **MORELLE ET AL.: "QUE PEUVENT NOUS APPORTER LES EXTRAITS VEGETAUX? 3eme PARTIE: LE GINSENG" PARFUMS COSMETIQUES AROMES, vol. 97, mars 1991, pages 91-103, XP000201984 PARIS FR**
- **PATENT ABSTRACTS OF JAPAN vol. 7, no. 213 (C-187) [1358] & JP 58 113116 A (MUTSUKO KURITA)**
- **PATENT ABSTRACTS OF JAPAN vol. 12, no. 482 (C-553) [3329] & JP 63 198609 A (EIKOUDOU KK)**
- **PATENT ABSTRACTS OF JAPAN vol. 4, no. 190 (C-037) & JP 55 129228 A (OSAKA CHEM LAB)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 001 740 B1

## Description

L'invention concerne de nouvelles utilisations du ginsenoside $Rb_1$ comme agent destiné à stimuler la synthèse de l'élastine.

Cette invention trouve des applications essentiellement dans le domaine de la cosmétique et de la pharmacie, notamment de la dermatologie.

D'une façon générale, lorsque l'on cherche à lutter efficacement contre le vieillissement cutané, on essaie de traiter la peau par des produits présentant une activité anti-radicalaire, susceptibles en particulier de piéger les radicaux libres responsables, pour partie, des effets néfastes entraînant un vieillissement de la peau.

Il est connu que dans le derme, les fibres élastiques forment un réseau qui contribue à l'élasticité de la peau et par conséquent à sa tonicité et à sa fermeté. L'élastine est le composant majeur de ces fibres. Elle est initialement synthétisée par les fibroblastes sous la forme d'un polypeptide soluble de 70 kDa, la tropoélastine. La formation des microfibrilles se réalise ensuite par des liaisons intramoléculaires appelées desmosines entre les résidus lysine des chaînes polypeptidiques. Ces liaisons confèrent à l'élastine une grande insolubilité.

Par ailleurs, il est connu que la teneur en élastine baisse dans l'organisme humain à partir de l'âge de 25 ans. Il y a de bonnes raisons de penser que l'apparition des rides peut être, entre autres, liée à la disparition progressive de l'élastine dermique. C'est pourquoi certains cosmétologues ont proposé de traiter la peau par des préparations de produits dérivés d'élastine. On a ainsi proposé d'utiliser des compositions contenant de l'élastine hydrolysée pour la rendre soluble. Toutefois, l'inconvénient de tels produits est lié au poids moléculaire trop élevé de l'élastine hydrolysée qui pénètre difficilement à travers la peau.

Une autre solution beaucoup plus intéressante, et constituant un net progrès, est de favoriser la synthèse d'élastine directement par les cellules de la peau. Deux types de produits sont à ce jour connus pour avoir un tel effet. Il s'agit d'une part de l'éthocyne dont l'activité a été décrite par L.C. FORD et al. (IFSCC Congres, Barcelona, 1986, Vol. II, p. 987-989) et d'autre part de l'acide ascorbique et de ses dérivés dont l'activité est décrite dans la demande internationale WO 96/19099.

La famille des Panax comprend de nombreuses espèces et variétés dont les plus connues sont :

- Panax ginseng (C.A. Meyer) qui est le plus fréquemment utilisé pour ses vertus médicales,
- Panax notoginseng,
- Panax pseudo-ginseng (subsp. himalaicus),
- Panax japonicus (var. major ; var. angustifolius),
- Panax quinquefolium,
- Panax trifolius,
- Panax zingiberensis,
- Panax stipuleanatus.

Les Panax proviennent essentiellement de trois pays :

- Japon
- Chine
- Corée.

Les espèces et variétés cultivées dans ces pays sont différentes et subissent des conditions géo-climatiques variées.

On constate des teneurs en saponines très différentes dans les diverses parties de la plante. La partie racinaire est la plus fréquemment utilisée et généralement réputée la plus active.

La teneur la plus forte en saponines est observée dans l'extrémité de la racine (ninjin, en japonais) et dans les radicelles (hairy roots : keninjin, en japonais).

Le Panax le plus connu est le Panax ginseng ou ginseng.

Il a une composition en saponines (appelées ginsenosides) bien particulière.

Chaque espèce ou variété de Panax a une composition en ginsenosides particulière, ce qui confère aux extraits qui en proviennent des propriétés différentes de celles des extraits d'autres Panax.

Le Panax notoginseng (Sanchi en chinois) et le Panax quinquefolium (American ginseng) différent beaucoup du Panax ginseng. Ils sont particutièrement riches en ginsenoside $Rb_1$ encore désigné par G-$Rb_1$ répondant à la formule :

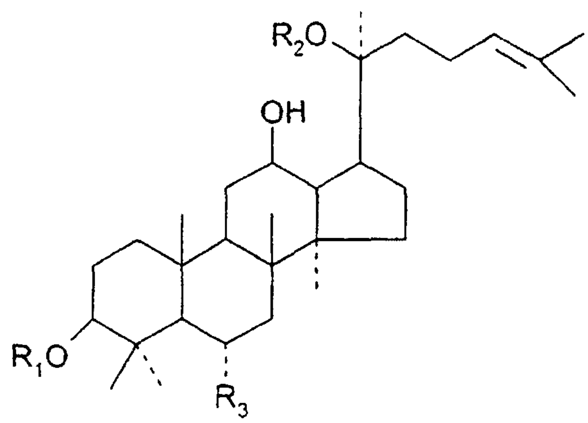

(I)

dans laquelle

$R_1$ = Glc(2-1)Glc,
$R_2$ = Glc(6-1)Glc,
$R_3$ = H

où Glc désigne un groupement β-D glucopyranosyle.

On a décrit dans la demande internationale PCT WO 94/06402 une activité de ce ginsenoside $Rb_1$ et, plus particulièrement, des extraits de panax notoginseng ou San-chi particulièrement riche en ce ginsenoside pour le soin des cheveux, en particulier pour éviter leur chute.

On connaît, par ailleurs, l'action inhibitrice de ce même ginsenoside sur la prolifération des kératinocytes. (Nack-in-Kim et al. J.Invest.Dermatol., 101 (3), 491, 1993.)

Ce même ginsenoside $Rb_1$ est, par ailleurs, particulièrement abondant dans les extraits de Panax quinquefolium.

Le Panax quinquefolium est surtout utilisé comme tonique (voir article référencé Hort. Technology 5 (1), 27-34, 1995 ).

Un autre ginsenoside, le ginsenoside $Rb_2$ a été trouvé efficace pour stimuler la synthèse des glycosaminoglycanes (GAG) par les fibrocytes (Tanaka H. et al., Nippon Koshohin Kagakkaishi, 15 (3), 132-5, 1991 et Tanaka H. et al. Fragrance J., 19 ( 8), 90-2, 1991).

Par ailleurs, des études ont montré que les ginsenosides de ginseng ont un effet positif sur l'élasticité de la peau des femmes ménopausées (A. Gezzi et al. Fitoterapia, 57 (1), 15-28, 1986. S.B. Curri et al. Fitoterapia, 57, 4, 217-222, 1986). Parallèlement, ces auteurs ont montré un effet positif d'un extrait de ginseng à 14 % de ginsenosides sur l'hydratation de la couche cornée de la peau des femmes. Dans le cas de l'extrait de ginseng utilisé qui contient d'autres ginsenosides comme saponines majeures et dans lequel le ginsenoside $Rb_1$ est en faible concentration, l'effet principal semble être un effet d'hydratation qui se traduit par une meilleure élasticité.

Par ailleurs, le brevet russe SU 839542 décrit une crème cosmétique contenant, entre autres, un extrait de ginseng. Cette crème est destinée à prévenir le flétrissement de la peau. Cette crème améliore la circulation sanguine, le métabolisme des protéines et des graisses, la structure morphologique de la peau et son élasticité.

Les inventeurs de la présente demande ont maintenant mis en évidence l'effet spécifique du ginsenoside $Rb_1$ encore dénommé G-$Rb_1$ pour stimuler la biosynthèse de l'élastine par les fibroblastes du derme, en particulier les fibroblastes du derme humain.

Ainsi, la présente invention a pour but de résoudre le nouveau problème technique consistant en la fourniture d'une solution permettant d'améliorer l'élasticité cutanée en favorisant la synthèse d'élastine, selon une solution de conception simple, aisément utilisable à l'échelle industrielle tout en permettant un usage cosmétique ou pharmaceutique, notamment dermatologique.

Selon l'une de ses caractéristiques essentielles, l'invention concerne l'utilisation cosmétique du ginsenoside $Rb_1$ (ci-après dénommé également G-$Rb_1$) ou d'extraits végétaux en contenant comme agent actif d'une composition destinée à stimuler la synthèse d'élastine par les fibroblastes du derme.

Selon une autre caractéristique essentielle, l'invention concerne l'utilisation du même ginsenoside $Rb_1$ ou d'extraits végétaux en contenant pour la fabrication d'une composition pharmaceutique, notamment dermatologique, destinée au traitement des troubles liés à une insuffisance de la synthèse de l'élastine par les fibroblastes du derme.

Dans ces deux types d'utilisation, la stimulation de la synthèse de l'élastine a pour résultat d'améliorer l'élasticité et la tonicité cutanée ou de favoriser le raffermissement de la peau.

Selon une variante particulièrement avantageuse de l'invention, les compositions aussi bien cosmétiques que pharmaceutiques, notamment dermatologiques, de l'invention pourront comprendre, outre le ginsenoside $Rb_1$, au moins une autre saponine de type ginsenoside.

Ainsi, selon une variante particulièrement avantageuse de l'invention, le ginsenoside $Rb_1$ pourra être introduit dans la composition sous la forme d'un extrait d'une plante de type Panax.

A titre d'exemple d'extrait particulièrement utile pour la mise en oeuvre de l'invention, on citera les extraits contenant :

- de 2 à 60 % en poids de saponine G-$Rb_1$,
- de 2 à 60 % en poids de saponine G-Rg1,
- de 0 à 15 % en poids de saponine G-Rd,
- de 0 à 15 % en poids de saponine N-$R_1$,
- de 1 à 10 % en poids de saponine G-Re.

On choisira de préférence encore un extrait contenant :

- de 10 à 60 % en poids de saponine G-$Rb_1$.
- de 10 à 60 % en poids de saponine G-Rg1,
- de 0 à 15 % en poids de saponine G-Rd,
- de 0 à 15 % en poids de saponine N-$R_1$,
- de 1 à 10 % en poids de saponine G-Re,

par rapport au poids total dudit extrait.

Selon une autre variante encore, on choisira un extrait dans lequel le ginsenoside G-$Rb_1$, est la saponine contenue majoritairement.

Comme on l'a vu précédemment, les plantes de type Panax notoginseng et Panax quinquefolium sont deux plantes de type Panax particulièrement riches en ginsenoside $Rb_1$. C'est donc à ces deux plantes particulières que l'on recourra de préférence pour la préparation des compositions cosmétiques ou pharmaceutiques, notamment dermatologiques de l'invention.

Pour ces deux types de plantes, on utilisera de préférence pour la préparation de l'extrait, leurs parties souterraines.

L'extrait est avantageusement obtenu par extraction de la matière végétale avec un solvant polaire ou un mélange de solvants polaires.

Dans ce cadre, les procédures d'extraction classiques bien connues de l'homme de l'art peuvent être utilisées. En particulier, l'extraction peut être réalisée sur la matière végétale broyée.

Avantageusement, l'extraction est réalisée à partir des rhizomes.

L'extraction peut également être réalisée sur des tissus végétaux en culture (culture in vitro de racines ou de cals).

La matière végétale broyée est introduite dans le solvant d'extraction, de préférence constitué par le solvant ou le mélange de solvants polaires précités. L'extraction peut être renouvelée plusieurs fois jusqu'à épuisement de la matière, conformément aux procédés bien connus de l'homme de l'art. L'extraction peut être réalisée à la température ambiante, ou à chaud et notamment au reflux du solvant. La proportion en poids entre la matière à extraire et le solvant peut varier dans de larges limites. Plus précisément, elle peut être comprise entre 1 : 5 et 1 : 100, et, de préférence, entre 1 : 10 et 1: 20.

Selon un mode particulier de réalisation de l'invention, l'extrait végétal contenant les saponines précitées est obtenu selon le procédé décrit ci-après à titre indicatif, mais nullement limitatif. On extrait la matière sèche au moyen d'un solvant choisi dans le groupe constitué par : l'eau, les alcools comportant de préférence de 1 à 4 atomes de carbone, et les solvants mixtes à base de mélanges quelconques des solvants précités.

De préférence, on utilisera un alcool tel que le méthanol, l'éthanol, le propanol, l'isopropanol, le propylèneglycol ou le butylèneglycol, ou un mélange de ces alcools ou encore un mélange hydro-alcoolique.

On choisira avantageusement comme solvant pour réaliser cette extraction un solvant choisi dans le groupe constitué par le méthanol, l'éthanol, le 1,3 -bulylèneglycol et les mélanges de ces solvants entre eux ou avec de l'eau.

De préférence, une première extraction dite extraction primaire est effectuée au reflux sous pression atmosphérique pendant une durée de 30 minutes environ. En fin d'extraction, on laisse refroidir la phase du solvant contenant l'extrait puis on la filtre. Avantageusement, le résidu est extrait encore une ou deux fois selon le même protocole, les filtrats sont réunis puis concentrés et ou évaporés à sec sous pression réduite. On obtient ainsi un premier extrait selon l'invention riche en saponines. Cet extrait peut également être lyophilisé.

Suivant une variante particulière, on prépare un mélange de saponines selon l'invention à partir du premier extrait concentré ou sec précité en opérant comme indiqué ci-après. Le premier extrait précité, qui a été obtenu de préférence avec un solvant primaire facilement éliminable par évaporation, tel que le méthanol ou l'éthanol ou encore un mélange méthanol-eau ou éthanol-eau, est introduit puis agité dans un solvant apolaire de préférence miscible avec le solvant d'extraction primaire, tel qu'un éther ou une cétone de faible poids moléculaire, en particulier l'éther éthylique ou isopropylique, l'acétone ou la méthyl-éthyl-cétone. La quantité de solvant apolaire est, en poids, généralement de 5 à 100 parties pour une partie d'extrait primaire. L'insoluble et/ou le précipité formé contient principalement un mélange de saponines utilisable selon l'invention.

Si on le désire, le mélange de saponines obtenu précédemment peut être purifié une nouvelle fois par un procédé quelconque à la portée de l'homme de l'art.

En particulier l'insoluble et/ou le précipité précité est remis en solution dans 20 fois environ son poids d'eau. La solution aqueuse est ensuite extraite 3 à 4 fois par un alcool peu soluble dans l'eau, tel que le butanol saturé en eau, par exemple en proportion 1/1 en volume à chaque opération d'extraction. Les phases alcooliques sont réunies et évaporées sous pression réduite. Le résidu est dissous dans 10 fois environ son poids d'eau, la solution est ensuite dialysée contre de l'eau pure pendant 4 à 5 jours. Le contenu de la cellule de dialyse est lyophilisé. Eventuellement pour améliorer la purification du mélange de saponines obtenu, le lyophilisat est dissous dans du méthanol, puis jeté dans de l'éther éthylique. On recueille le précipité formé.

Selon un autre mode de réalisation avantageux, le ginsenoside $Rb_1$ ou les extraits précités sont utilisés de préférence par voie topique à une concentration comprise entre 0,001 % et 5% par rapport au poids total d'une composition le contenant dans un excipient, véhicule ou support approprié, de préférence cosmétiquement ou pharmaceutiquement, notamment dermatologiquement, acceptable. Une concentration préférée est comprise entre 0.01 et 1% en poids par rapport au poids total de la composition le contenant.

Selon un mode de réalisation avantageux de l'invention, la composition cosmétique ou pharmaceutique, notamment dermatologique de l'invention contient, en outre, de l'acide ascorbique, en particulier de l'acide L-ascorbique ou vitamine C ou son isomère, l'acide érythorbique ou un de leurs sels ou esters, désignés sous le nom générique de dérivés ascorbiques.

Selon un mode de réalisation avantageux de l'invention, le ginsenoside $Rb_1$ ou les extraits selon l'invention sont utilisés en combinaison avec une quantité efficace d'un principe actif stimulant la synthèse des composants de la matrice extracellulaire du derme, parmi lesquels on citera le collagène, en particulier le collagène I et le collagène III, et les glycosaminoglycanes, en particulier l'acide hyaluronique.

On utilisera avantageusement, à titre de principe actif stimulant la synthèse du collagène, en particulier du collagène I et du collagène III, le madécassoside, un extrait de Centella asiatica, ou un extrait de ginseng, en particulier un extrait contenant le ginsenoside $R_O$, ledit principe actif étant à une concentration comprise entre 0,01 % et 5 % en poids par rapport au poids total de la composition finale.

On utilisera, à titre d'exemple de principe actif stimulant la synthèse des glycosaminoglycanes, un extrait végétal tel qu'un extrait de Filicium decipiens, en particulier un extrait d'écorce de racine de cette plante, décrit dans la demande de brevet français 95 07708, ou un extrait d'Eryobotria japonica décrit dans la demande de brevet français 96. 00018, ou un facteur de croissance, en particulier le PDGF (platelet derived growth factor), facteur de croissance dérivé des plaquettes sanguines.

Selon un autre mode de réalisation avantageux, le ginsenoside $Rb_1$ ou les extraits précités sont utilisés en combinaison avec une vitamine, en particulier la vitamine A, son ester palmitique, propionique ou acétique ou la vitamine E et ses dérivés, en particulier à une concentration comprise entre 0.0001 et 5 % en poids par rapport au poids total de la composition.

Selon un troisième aspect, la présente invention couvre aussi une méthode de traitement, cosmétique ou thérapeutique, de la peau, destinée à prévenir ou à corriger une perte d'élasticité, de tonicité ou de fermeté cutanée, caractérisée en ce qu'elle comprend l'application sur les zones de la peau à traiter d'une quantité efficace du ginsenoside $Rb_1$ ou d'un extrait en contenant, pour stimuler la synthèse d'élastine par les fibroblastes, ledit produit étant incorporé dans un excipient cosmétiquement ou pharmaceutiquement acceptable.

Pour la mise en oeuvre d'un tel procédé, on choisira avantageusement un extrait de parties souterraines de Panax notoginseng ou de Panax quinquefolium.

Les variantes de réalisation de cette méthode résultent également clairement des variantes de réalisation de l'utilisation précitée.

Il résulte de ce qui précède que le ginsenoside $Rb_1$ ou l'extrait en contenant sont précieux pour la fabrication d'une composition cosmétique ou pharmaceutique, notamment dermatologique, où l'on recherche une amélioration de l'élasticité cutanée par stimulation de la synthèse de l'élastine.

L'homme de l'art comprend ainsi l'intérêt majeur de l'invention qui favorise la synthèse d'élastine et sa facilité d'emploi grâce à une application topique sur la peau.

D'autres buts, caractéristiques et avantages de l'invention apparaîtront clairement à la lumière de la description explicative qui va suivre faite en référence aux exemples donnés ci-après simplement à titre d'illustration et qui ne sauraient donc en aucune façon limiter la portée de l'invention.

Dans les exemples, tous les pourcentages sont donnés en poids sauf indication contraire.

## EXEMPLES

Exemple 1

Préparation d'un extrait de Panax notoginseng

200 g de racines séchées de Panax notoginseng (San-Chi) sont broyés, puis extraits par 2 litres de méthanol à reflux pendant 30 minutes. On laisse refroidir la phase méthanolique jusqu'à température ambiante, puis on filtre. Le résidu solide est extrait une nouvelle fois, de la même façon, avec 2 litres de méthanol.

On réunit les deux filtrats méthanoliques, et on évapore le solvant sous vide jusqu'à l'obtention d'un résidu solide.

Ce solide est ensuite traité par 100 ml d'acétone. La partie insoluble dans l'acétone est récupérée et mise en solution dans 50 ml de méthanol. On ajoute alors à cette solution de l'éther éthylique jusqu'à l'obtention d'un précipité.

Ce précipité est filtré. Il constitue un mélange de saponines de Panax notoginseng selon l'invention, contenant en particulier le ginsenoside G-$Rb_1$. Ce mélange, désigné extrait $E_1$, sera mis en oeuvre dans les essais rapportés à l'exemple 3 ci-après (voir tableau 2).

Exemple 2

Préparation d'extraits de Panax quinquefolium

a) Préparation d'un extrait méthanolique

200 g de racines séchées de Panax quinquefolium (Ginseng américain) sont broyés, puis extraits par 2 litres de méthanol à reflux pendant 30 minutes.

Comme dans l'exemple précédent, on laisse refroidir la phase méthanolique, puis on recommence deux fois l'opération d'extraction.

On réunit ensuite les différents filtrats et on évapore à sec sous vide dans un ballon rotatif.

On obtient ainsi un extrait méthanolique de Panax quinquefolium riche en saponines, contenant en particulier le ginsenoside G-$Rb_1$. Cet extrait, dénommé extrait $E_2$, sera mis en oeuvre dans les essais rapportés à l'exemple 3 ci-après (voir tableau 3).

b) Préparation d'un mélange de saponines extrait du Panax quinquefolium

L'extrait $E_2$ obtenu précédemment est traité par 100 ml d'acétone. La partie insoluble est filtrée et dissoute dans 50 ml de méthanol. On ajoute à la solution obtenue de l'éther éthylique jusqu'à formation d'un précipité que l'on filtre et que l'on sèche.

Le résidu solide (dénommé $E_3$) obtenu est constitué essentiellement d'un mélange de saponines du Panax quinquefolium, contenant en particulier le ginsenoside G-$Rb_1$. Cet extrait est lui aussi mis en oeuvre dans les essais rapportés à l'exemple 3 (voir tableau 3).

c) Préparation d'une fraction d'extrait de Panax quinquefolium, riche en ginsenoside G-$Rb_1$

Le mélange de saponines obtenu à l'étape précédente est dissous dans 10 ml d'eau. Cette solution est adsorbée sur 10 g de Célite que l'on laisse sécher sous une hotte. Lorsque la poudre est sèche, on la place en haut d'une colonne de silice. On réalise alors une chromatographie suivant les techniques habituelles bien connues de l'homme de l'art. On élue ainsi successivement les saponines avec un mélange chloroforme-méthanol-eau 50/50/3. On recueille différentes fractions, et on choisit la fraction la plus riche en ginsenoside G-$Rb_1$ en réalisant des chromatographies sur couche mince en comparaison avec un témoin du commerce.

Cette fraction, dénommée $E_4$, riche en ginsenoside G-$Rb_1$, est mise en oeuvre dans les essais rapportés à l'exemple 3 ci-après (voir tableau 3).

Exemple 3

Exemple d'essai démontrant l'activité du ginsenoside G-$Rb_1$ et d'extraits en contenant sur la stimulation de la synthèse de l'élastine par des fibroblastes cutanés humains

Matériel et méthodes

Provenance des cellules :

On utilise dans cette étude une souche de fibroblastes humains normaux (FHN) provenant de peau chirurgicale du visage d'une femme de 50 ans (FHN-50 ans) et d'une femme de 47 ans (FHN-47 ans). Les cellules avaient été obtenues par la méthode des explants telle que décrite par Dumas M. et al. dans l'article ayant pour titre "in vitro biosynthesis of type I and III collagens by human dermal fibroblasts from donors of increasing ages" dans Mech. Ageing Dev. 73 (1994), pages 179-187.

Mise en oeuvre des cultures de cellules :

Ces fibroblastes cutanés humains normaux ainsi obtenus sont ensemencés dans les puits d'une boîte de culture multipuits, dans un milieu de culture E 199, dans lequel on a ajouté 2 millimoles par litre de L-glutamine.

De plus, pour un certain nombre d'essais, le milieu de culture contient de l'ascorbate de sodium aux concentrations de 15 ou 25 micromoles par litre.

24 heures après l'ensemencement, certains puits reçoivent le ginsenoside $Rb_1$ ou un extrait de Panax de 1 à 10µg/ml par l'intermédiaire d'une solution dans le DMSO de façon à ce que la concentration finale de DMSO soit de 0,1 %.

On utilise à chaque fois six puits par produit et par concentration, ainsi que six puits ne recevant aucun produit à tester pour les cultures témoins. Ces cultures témoins ne reçoivent que du DMSO à une concentration finale de 0,1 % en poids.

La culture est poursuivie pendant 48 h.

Après 48 h de culture comme indiqué ci-dessus, on dose sur les surnageants de la culture l'élastine sécrétée, par une méthode ELISA dérivée de celle utilisée pour le dosage du collagène, comme décrit par Dumas M. et al. dans la publication citée ci-dessus, à ceci près qu'un anticorps polyclonal anti-élastine humaine du commerce a été utilisé à la place de l'anticorps polyclonal anti-collagène I et III humain.

Ce dosage est naturellement réalisé aussi sur des cultures témoins, ne recevant pas de produit à tester.

Une gamme étalon réalisée à partir d'élastine soluble du commerce permet de convertir en nanogramme d'élastine les valeurs de densité optique (DO) obtenues à partir des surnageants de culture.

Dosage des protéines :

Parallèlement au dosage de l'élastine, un dosage de protéines cellulaires est effectué sur les cultures de fibroblastes, après élimination du surnageant, afin de ramener la quantité d'élastine dosée à une quantité fixe de protéines cellulaires. Le dosage est effectué en utilisant le kit BCA-1 (Bicinchoninic acid kit, commercialisé par Sigma, France) après dissolution du tapis cellulaire par de la soude 0,1 N.

Expression des résultats et statistiques :

Les résultats sont exprimés en nanogrammes d'élastine sécrétée par microgramme de protéines cellulaires. L'activité A de stimulation, exprimée en pourcentage, est calculée selon la formule suivante :

$$A = \frac{q - q_o}{q0} x\ 100$$

dans laquelle : q représente la quantité d'élastine sécrétée par les FHN traités,

$q_o$ représente la quantité d'elastine sécrétée par les FHN témoins.

Les résultats obtenus sur les cultures traitées (n=6) et témoins (n=6) sont comparés à l'aide du test t de Student pour séries non appariées. Toute valeur de $p < 0.05$ indiquera qu'une différence significative existe entre la sécrétion d'élastine des FHN témoins et ceux traités avec les produits de l'invention.

Les tableaux 1A, 1B, 2 et 3 ci-dessous rapportent les résultats obtenus avec le ginsenoside $Rb_1$ et les diffé-

rents extraits $E_1$, $E_2$, $E_3$ et $E_4$ obtenus selon les exemples 1 et 2.

Plus précisément :

- le tableau 1A donne les résultats obtenus avec un ginsenoside $Rb_1$ commercial introduit dans des cultures de fibroblastes du visage d'une femme de 47 ans (FHN-47 ans),
- le tableau 1B donne les résultats obtenus avec le même ginsenoside commercial introduit dans des cultures de fibroblastes du visage d'une femme de 50 ans (FHN-50 ans),
- le tableau 2 donne les résultats obtenus avec les extraits de saponines de Panax notoginseng obtenus selon l'exemple 1 ($E_1$), introduits dans des cultures de fibroblastes du visage d'une femme de 50 ans (FHN-50 ans),
- le tableau 3 donne les résultats obtenus avec les extraits de Panax quinquefolium (notés $E_{2,}$ $E_3$ et $E_{4)}$ obtenus selon l'exemple 2.

TABLEAU 1A

| **ACTIVITE DU GINSENOSIDE $Rb_1$ SUR LA SYNTHESE D'ELASTINE (FHN-47 ans)** | | | |
|---|---|---|---|
| Conditions expérimentales | ng Elastine/ µg prot/48 h | Activité en % | Test t de Student valeurs de p |
| T. + DMSO 0,1 % | 19,7 ± 1,6 | 0 | |
| G-$Rb_1$ 10 µg/ml | 25,8 ± 2,2 | 31 | 0,00033 |
| T. + DMSO 0,1 % + Asc. 15 µM | 33,9 ± 1,9 | 0 | |
| G-$Rb_1$ 10 µg/ml + Asc. 15 µM | 39,3 ± 2,8 | 16 | 0,0039 |
| T. + DMSO 0,1 % + Asc. 25 µM | 35, 1± 2,9 | 0 | |
| G-$Rb_1$ 10 µg/ml + Asc. 25 µM | 38,8 ± 6,8 | 10 | 0,2663 |
| T. = Témoin; Asc. = Ascorbate | | | |

TABLEAU 1B

| **ACTIVITE DU GINSENOSIDE $Rb_1$ SUR LA SYNTHESE D'ELASITNE (FHN-50 ans)** | | | |
|---|---|---|---|
| Conditions expérimentales | ng Elastine/ µg prot/48 h | Activité en % | Test t de Student valeurs de p |
| T. + DMSO 0,1% + Asc. 25 µM | 23,9 ±2,4 | 0 | |
| G-$Rb_1$ 10 µg/ml - Asc. 25 µM | 28 ± 2,2 | 17 | 0,0125 |
| T. + DMSO 0.1 % + Asc. 25 µM | 21,7 ± 1,2 | 0 | |
| G-$Rb_1$ 10 µg/ml +Asc. 25µM | 26,3 ± 3,7 | 21 | 0,0154 |
| T. + DMSO 0.1 % + Asc. 25 µM | 27,3 ± 2,6 | 0 | |
| G-$Rb_1$ 10 µg/ml + Asc. 25 µM | 32 ± 4,3 | 17 | 0,0449 |
| T. = Témoin ; Asc. = Ascorbate | | | |

TABLEAU 2

| **ACTIVITE D'UN EXTRAIT D'UN EXTRAIT DE PANAX NOTOGINSENG SUR LA SYNTHESE DE L'ELASTINE (FHN-50 ans)** | | | |
|---|---|---|---|
| Conditions expérimentales | ng Elastine/ µg prot/48 h | Activité en % | Test t de Student valeurs de p |
| T. + DMSO 0,1 % + Asc. 25 µM | 33,4 ± 4,3 | 0 | |
| $E_1$ 1 µg/ml - Asc. 25 µM | 38,8 ± 3,4 | 16 | 0,038 |
| $E_1$ 2,5 µg/ml + Asc. 25 µM | 39,7 ± 2,9 | 19 | 0,015 |
| T. + DMSO 0,1 % - Asc. 25 µM | 32,2 ± 7,4 | 0 | |
| $E_1$ 1 µg/ml + Asc. 25 µM | 35,6 ± 5,9 | 11 | 0,404 |
| $E_1$ 2,5 µg/ml - Asc. 25 µM | 40,9 ± 4,7 | 27 | 0,0362 |
| T. = Témoin<br>Asc. = Ascorbate | | | |

TABLEAU 3

| ACTIVITE D'EXTRAITS DE PANAX QUINQUEFOLIUM SUR LA SYNTHESE DE L'ELASTINE (FHN-50 ans) | | | |
|---|---|---|---|
| Conditions expérimentales | ng Elastine/ µg prot/48 h | Activité en % | Test t de Student valeur de p |
| T. + DMSO 0,1 % + Asc. 25 µM | 21,7 ± 1,2 | 0 | |
| $E_2$ 1 µg/ml + Asc. 25 µM | 22,9± 2,5 | 5,5 | 0,33 |
| $E_2$ 2.5 µg/ml + Asc 25 µM | 25,2 ± 3,5 | 16 | 0,044 |
| T. + DMSO 0,1 % + Asc. 25 µM | 23,6 ± 1,7 | 0 | |
| $E_3$ 1 µg/ml + Asc. 25 µM | 24,3 ± 1,7 | 3 | 0,473 |
| $E_3$ 2,5 µg/ml +Asc. 25 µM | 29,4 ± 4,2 | 25 | 0,01 |
| Asc. 25 µM + $E_4$ 1 µg/ml | 27,9 ± 1,1 | 18 | 0,0004 |
| T. + DMSO 0,1% + Asc. 25 µM | 33,4 ± 4,3 | 0 | |
| $E_2$ 1 µg/ml + Asc. 25 µM | 35,3 ± 6 | 5,7 | 0,546 |
| $E_2$ 2,5 µg/ml + Asc. 25 µM | 41,9 ± 3,8 | 25 | 0,005 |
| T. + DMSO 0,1 % + Asc. 25 µM | 27,3 ± 2,6 | 0 | |
| $E_3$ 1 µg/ml + Asc. 25 µM | 29,7 ± 4,1 | 8,8 | 0,239 |
| $E_3$ 2,5 µg/ml + Asc. 25 µM | 31,4 ± 2,9 | 15 | 0,025 |
| Asc. 25 µM + $E_4$ 1 µg/ml | 30,7 ± 3,7 | 12 | 0,094 |
| T. = Témoin ; Asc. = Ascorbate | | | |

Conclusion

Comme il ressort des tableaux 1 à 3, le ginsenoside $Rb_1$, les extraits de Panax notoginseng ainsi que les extraits de Panax quinquefolium stimulent fortement la synthèse d'élastine par les fibroblastes. Cette stimulation augmente avec la concentration du produit testé.

Il est donc démontré que l'utilisation du ginsenoside $Rb_1$, d'un extrait de Panax notoginseng ou d'un extrait de Panax quinquefolium, permet de stimuler la synthèse d'élastine par les fibroblastes de manière significative, et particulièrement inattendue.

Exemples d'applications

Les compositions sont exprimées ci-dessous en pourcentages en poids.

Exemple 4

Gel pour le soin du visage :

| | |
|---|---|
| ginsenoside $Rb_1$ | 0,2 |
| lécithine hydrogénée | 1 |
| acide hyaluronique | 1 |
| Carbopol 941® | 1,25 |
| eau + conservateur + triéthanolamine | qsp 100. |

En application locale 2 fois par jour sur le bas du visage et le cou, ce produit de soin améliore et maintient l'élasticité de la peau du visage et en atténue les rides et ridules.

Exemple 5

Crème raffermissante pour le visage:

| | |
|---|---|
| propylèneglycol | 1 |
| glycérylstéarate PEG 100 | 2 |
| glycérylstéarate sodium lauryl sulfate | 4 |
| glyceryl stéarate | 3 |
| alcool cétylique | 1 |
| triglycéride caprilique/capric | 3 |
| octyl stéarate | 2 |
| huile d'avocat | 1 |
| huile de camélia | 1 |
| palmitate d'ascorbyle | 0,5 |
| palmitate de vitamine A | 0,01 |
| glycocéramides de blé | 0,1 |
| acide lactique | 0,5 |
| Carbopol 940® | 0,5 |
| saponines de Panax notoginseng ($E_1$) | 0,5 |
| eau + conservateur + parfums | qsp 100. |

Cette crème s'applique matin et soir et redonne l'élasticité à la peau du visage qui présente alors un meilleur aspect.

Exemple 6

Lait de soin corporel :

| | |
|---|---|
| huile végétale | 2 |
| triglycéride caprylic/capric | 5 |
| acétate de vitamine E | 0,56 |
| carbomer | 0,5 |
| gomme xanthane | 0,20 |
| acide hyaluronique | 0,15 |
| extrait de Panax quinquefolium ($E_2$) | 0,5 |
| extrait de centella asiatica | 0,1 |
| extrait de thé vert | 0,05 |
| eau + conservateur + agent rafraîchissant + parfum | qsp 100. |

En application sur le buste le soir par cure de 20 jours, avec massage pour permettre aux tissus du buste de conserver leur élasticité.

Exemple 7

Lotion pour améliorer l'élasticité de la peau du visage

| | |
|---|---|
| Saponines de Panax quinquefolium ($E_3$)<br>solution aqueuse à 1 % d'acide hyaluronique<br>glycérol<br>eau + conservateur | 0,3<br>1<br>2<br>qsp 100. |

Cette lotion, pour améliorer l'élasticité de la peau, peut être utilisée après le démaquillage pour les femmes ou après le rasage pour les hommes.

L'invention couvre également tous les équivalents techniques des moyens décrits ainsi que leurs diverses combinaisons.

**Revendications**

1. Utilisation cosmétique du ginsenoside $Rb_1$ ($G\text{-}Rb_1$) ou d'extraits végétaux en contenant comme agent actif stimulant la synthèse de l'élastine par les fibroblastes du derme, ledit agent actif étant incorporé dans une composition cosmétique comprenant un véhicule cosmétiquement acceptable.

2. Utilisation du ginsénoside $Rb_1$ ($G\text{-}Rb_1$) ou d'extraits végétaux en contenant pour la fabrication d'une composition pharmaceutique, notamment dermatologique, destinée au traitement des troubles liés à une insuffisance de la synthèse de l'élastine par les fibroblastes du derme.

3. Utilisation selon l'une des revendications 1 ou 2, **caractérisée en ce que** ladite composition contient de 0,001 à 5 % en poids dudit ginsenoside ou dudit extrait.

4. Utilisation selon l'une des revendications 1 à 3, **caractérisée en ce que** ladite composition contient, outre le ginsenoside $Rb_1$, au moins une autre saponine de type ginsenoside.

5. Utilisation selon l'une des revendications 1 à 4, **caractérisée en ce que** ledit ginsenoside $G\text{-}Rb_1$ est introduit dans ladite composition sous la forme d'un extrait d'une plante de type Panax.

6. Utilisation selon la revendication 5, **caractérisée en ce que** ledit extrait contient :

   - de 2 à 60 % en poids de saponine $G\text{-}Rb_1$,
   - de 2 à 60 % en poids de saponine G-Rg1,
   - de 0 à 15 % en poids de saponine G-Rd,
   - de 0 à 15 % en poids de saponine $N\text{-}R_1$,
   - de 1 à 10 % en poids de saponine G-Re,

   par rapport au poids total dudit extrait.

7. Utilisation selon l'une des revendications 5 ou 6, **caractérisée en ce que** ledit extrait contient :

   - de 10 à 60 % en poids de saponine $G\text{-}Rb_1$,
   - de 10 à 60 % en poids de saponine $G\text{-}Rg_1$,
   - de 0 à 15 % en poids de saponine G-Rd,
   - de 0 à 15 % en poids de saponine $N\text{-}R_1$,
   - de 1 à 10 % en poids de saponine G-Re,

   par rapport au poids total de l'extrait.

8. Utilisation selon l'une des revendications 5 à 7, **caractérisée en ce que** ledit ginsenoside $G\text{-}Rb_1$ est la saponine contenue majoritairement dans ledit extrait.

9. Utilisation selon l'une des revendications 5 à 8, **caractérisée en ce que** ladite plante de type Panax est le Panax notoginseng ou le Panax quinquefolium.

**10.** Utilisation selon l'une des revendications 5 à 9, **caractérisée en ce que** ledit extrait est un extrait de Panax notoginseng, de préférence de parties souterraines de la plante.

**11.** Utilisation selon l'une des revendications 5 à 9, **caractérisée en ce que** ledit extrait est un extrait de Panax quinquefolium, de préférence un extrait d'une partie souterraine de cette plante.

**12.** Utilisation selon l'une des revendications 5 à 11, **caractérisée en ce que** ledit extrait est obtenu par extraction au moyen d'un solvant polaire ou d'un mélange de solvants polaires.

**13.** Utilisation selon l'une des revendications 5 à 12, **caractérisée en ce que** ledit extrait est obtenu par extraction au moyen d'un solvant ou d'un mélange de solvants choisis dans le groupe constitué par l'eau, les alcools comportant de préférence de 1 à 4 atomes de carbone, en particulier le méthanol, l'éthanol, le propanol, l'isopropanol, le propylèneglycol et le butylèneglycol.

**14.** Utilisation selon la revendication 13, **caractérisée en ce que** le solvant ou mélange de solvants est choisi dans le groupe constitué par le méthanol, l'éthanol, le 1,3-butylèneglycol et les mélanges de ces solvants entre eux ou avec de l'eau.

**15.** Utilisation selon l'une des revendications 1 à 14, **caractérisée en ce que** ladite composition contient, en outre, de l'acide ascorbique, en particulier de l'acide L-ascorbique ou vitamine C ou son isomère l'acide érythorbique ou un de leurs sels ou esters, désignés sous le nom générique de dérivés ascorbiques.

**16.** Utilisation selon l'une des revendications 1 à 14, **caractérisée en ce que** ladite composition contient, en outre, une quantité efficace d'un principe actif stimulant la synthèse des composants de la matrice extracellulaire du derme, en particulier du collagène, en particulier du collagène I et du collagène III, et des glycosaminoglycanes.

**17.** Utilisation selon la revendication 16, **caractérisée en ce que** ladite composition contient un principe actif stimulant la synthèse du collagène, en particulier du collagène I et du collagène III, choisi dans le groupe constitué par le madécassoside, un extrait de centella asiatica et un extrait de ginseng, en particulier le ginsenoside Ro, à une concentration en poids de 0,01 % à 5 %.

**18.** Utilisation selon la revendication 16, **caractérisée en ce que** la composition contient un principe actif stimulant la synthèse des glycosaminoglycanes, choisi dans le groupe constitué par un extrait de Filicium décipiens, en particulier un extrait d'écorce de racine de cette plante, un extrait d'Eriobotrya japonica et le PDGF.

**19.** Utilisation selon l'une des revendications 1 à 18, **caractérisée en ce que** ladite composition contient, en outre, des vitamines, en particulier de la vitamine A, son ester palmitique, propionique ou acétique ou la vitamine E et ses dérivés, à une concentration en poids de 0,0001 à 5 % par rapport au poids total de ladite composition.

**20.** Méthode de traitement cosmétique destinée à prévenir ou à corriger une perte d'élasticité, de tonicité ou de fermeté cutanée, **caractérisée en ce qu'**elle comprend l'application sur les zones de la peau à traiter d'une quantité efficace du ginsénoside $Rb_1$ ou d'un extrait végétal en contenant pour stimuler la synthèse de l'élastine par les fibroblastes du derme, ledit ginsenoside étant incorporé dans un excipient cosmétiquement acceptable.

## Patentansprüche

**1.** Kosmetische Verwendung des Ginsenosids $Rb_1$ ($G\text{-}Rb_1$) oder von dieses enthaltenden Pflanzenextrakten als Wirkmittel, das die Synthese des Elastins durch die Fibroblasten der Haut stimuliert, wobei das genannte Wirkmittel in einer kosmetischen Zusammensetzung enthalten ist, die ein kosmetisch geeignetes Trägermittel umfasst.

**2.** Verwendung des Ginsenosids $Rb_1$ ($G\text{-}Rb_1$) oder von dieses enthaltenden Pflanzenextrakten zur Herstellung einer pharmazeutischen und insbesondere einer dermatologischen Zusammensetzung zur Behandlung von Problemen, die mit einer ungenügenden Synthese des Elastins durch die Fibroblasten der Haut zusammenhängen.

**3.** Verwendung gemäß einem der Ansprüche 1 oder 2, **dadurch gekennzeichnet, dass** die genannte Zusammensetzung 0,001 bis 5 Gew.% des genannten Ginsenosids oder Extrakts enthält.

4. Verwendung gemäß einem der Ansprüche 1 bis 3, **dadurch gekennzeichnet, dass** die genannte Zusammensetzung, außer dem Ginsenosid $Rb_1$, mindestens ein weiteres Saponin vom Ginsenosid-Typ enthält.

5. Verwendung gemäß einem der Ansprüche 1 bis 4, **dadurch gekennzeichnet, dass** das genannte Ginsenosid G-$Rb_1$ in der genannten Zusammensetzung in Form eines Pflanzenextrakts vom Panax-Typ enthalten ist.

6. Verwendung gemäß Anspruch 5, **dadurch gekennzeichnet, dass** der genannte Extrakt enthält:

- 2 bis 60 Gew.% Saponin G-$Rb_1$,
- 2 bis 60 Gew.% Saponin G-$Rg_1$,
- 0 bis 15 Gew.% Saponin G-Rd,
- 0 bis 15 Gew.% Saponin N-$R_1$ ,
- 1 bis 10 Gew.% Saponin G-Re,

bezogen auf das Gesamtgewicht des Extrakts.

7. Verwendung gemäß einem der Ansprüche 5 oder 6,
**dadurch gekennzeichnet, dass** der genannte Extrakt enthält:

- 10 bis 60 Gew.% Saponin G-$Rb_1$,
- 10 bis 60 Gew.% Saponin G-$Rg_{1,}$
- 0 bis 15 Gew.% Saponin G-Rd,
- 0 bis 15 Gew.% Saponin N-$R_{1,}$
- 1 bis 10 Gew.% Saponin G-Re,

bezogen auf das Gesamtgewicht des Extrakts.

8. Verwendung gemäß einem der Ansprüche 5 bis 7, **dadurch gekennzeichnet, dass** das genannte Ginsenosid G-$Rb_1$ das Saponin ist, das überwiegend im genannten Extrakt enthalten ist.

9. Verwendung gemäß einem der Ansprüche 5 bis 8, **dadurch gekennzeichnet, dass** die genannte Pflanze vom Panax-Typ das Panax Notoginseng oder das Panax Quinquefolium ist.

10. Verwendung gemäß einem der Ansprüche 5 bis 9, **dadurch gekennzeichnet, dass** der genannte Extrakt ein Extrakt aus Panax Notoginseng, vorzugsweise aus den unterirdischen Teilen der Pflanze, ist.

11. Verwendung gemäß einem der Ansprüche 5 bis 9, **dadurch gekennzeichnet, dass** der genannte Extrakt ein Extrakt aus Panax Quinquefolium, vorzugsweise ein Extrakt aus einem unterirdischen Teil dieser Pflanze, ist.

12. Verwendung gemäß einem der Ansprüche 5 bis 11, **dadurch gekennzeichnet, dass** der genannte Extrakt durch Extraktion mit einem polaren Lösungsmittel oder einer Mischung aus polaren Lösungsmitteln erhalten wird.

13. Verwendung gemäß einem der Ansprüche 5 bis 12, **dadurch gekennzeichnet, dass** der genannte Extrakt durch Extraktion mit einem Lösungsmittel oder einer Mischung von Lösungsmitteln erhalten wird, ausgewählt aus der Gruppe aus Wasser und Alkoholen mit vorzugsweise 1 bis 4 Kohlenstoffatomen, insbesondere aus Methanol, Ethanol, Propanol, Isopropanol, Propylenglycol oder aus Butylenglycol.

14. Verwendung gemäß Anspruch 13, **dadurch gekennzeichnet, dass** das Lösungsmittel oder die Mischung von Lösungsmitteln aus der Gruppe aus Methanol, Ethanol, $_1$,3-Butylenglycol und aus den Mischungen dieser Lösungsmittel untereinander oder mit Wasser ausgewählt sind.

15. Verwendung gemäß einem der Ansprüche 1 bis 14, **dadurch gekennzeichnet, dass** die genannte Zusammensetzung außerdem Ascorbinsäure, insbesondere L-Ascorbinsäure oder Vitamin C oder sein Isomer Erythorbinsäure oder eines von deren Salzen oder Estern, die mit dem generischen Namen von Ascorbin-Derivaten bezeichnet werden, enthält.

16. Verwendung gemäß einem der Ansprüche 1 bis 14, **dadurch gekennzeichnet, dass** die genannte Zusammensetzung außerdem eine wirksame Menge eines Wirkprinzips enthält, das die Synthese der Bestandteile der extrazellulären Matrix der Haut, insbesondere des Kollagens, insbesondere des Kollagens I und III, und der Glycosaminoglycane, stimuliert.

**17.** Verwendung gemäß Anspruch 16, **dadurch gekennzeichnet, dass** die genannte Zusammensetzung ein Wirkprinzip, das die Synthese des Kollagens, insbesondere des Kollagens I und III, stimuliert, ausgewählt aus der Gruppe aus Madecassosid, einem Extrakt aus Centella asiatica und aus einem Extrakt aus Ginseng, insbesondere aus dem Ginsenosid Ro, mit einer Konzentration von 0,01 bis 5 Gew.% enthält.

**18.** Verwendung gemäß Anspruch 16, **dadurch gekennzeichnet, dass** die Zusammensetzung ein Wirkprinzip enthält, das die Synthese der Glycosaminoglycane stimuliert, ausgewählt aus der Gruppe aus einem Extrakt aus Filicium decipiens, insbesondere aus einem Extrakt aus der Wurzelrinde dieser Pflanze, einem Extrakt aus Eriobotrya japonica und aus dem PDGF.

**19.** Verwendung gemäß einem der Ansprüche 1 bis 18, **dadurch gekennzeichnet, dass** die genannte Zusammensetzung außerdem Vitamine, insbesondere Vitamin A, dessen Palmitin-, Propion- oder Essigsäureester oder Vitamin E und dessen Derivate in einer Konzentration von 0,0001 bis 5 Gew.%, bezogen auf das Gesamtgewicht der genannten Zusammensetzung, enthält.

**20.** Verfahren zur kosmetischen Behandlung zur Vorbeugung oder Korrektur eines Verlustes der Elastizität, Tonizität oder Straffheit der Haut, **dadurch gekennzeichnet, dass** man auf die zu behandelnden Zonen der Haut eine wirksame Menge des Ginsenosids $Rb_1$ oder eines dieses enthaltenden Pflanzenextrakts zur Stimulierung der Synthese des Elastins durch die Fibroblasten der Haut anwendet und aufbringt, wobei das genannte Ginsenosid in einem kosmetisch geeigneten Exzipient enthalten ist.

## Claims

**1.** Cosmetic use of ginsenoside $Rb_1$ ($G\text{-}Rb_1$) or of plant extracts containing same as active agent stimulating the synthesis of elastin by the fibroblasts of the dermis, said active agent being incorporated in a cosmetic composition comprising a cosmetically acceptable vehicle.

**2.** Use of ginsenoside $Rb_1$ ($G\text{-}Rb_1$) or of plant extracts containing same for the preparation of a pharmaceutical composition, notably a dermatological composition, intended for the treatment of the disorders linked to an insufficiency in the synthesis of elastin by the fibroblasts of the dermis.

**3.** Use according to one of claims 1 or 2, **characterised in that** said composition contains 0.001 to 5 % by weight of said ginsenoside or of said extract.

**4.** Use according to one of claims 1 to 3, **characterised in that** said composition contains, in addition to the ginsenoside $Rb_1$, at least one other saponin of the ginsenoside type.

**5.** Use according to one of claims 1 to 4, **characterised in that** said ginsenoside $G\text{-}Rb_1$ is introduced into said composition in the form of an extract of a plant of the Panax type.

**6.** Use according to claim 5, **characterised in that** said extract contains :

- 2 to 60 % by weight of saponin $G\text{-}Rb_1$,
- 2 to 60 % by weight of saponin G-Rg1,
- 0 to 15 % by weight of saponin G-Rd,
- 0 to 15 % by weight of saponin $N\text{-}R_1$,
- 1 to 10 % by weight of saponin G-Re,

with respect to the total weight of said extract.

**7.** Use according to one of claims 5 or 6, **characterised in that** said extract contains :

- 10 to 60 % by weight of saponin $G\text{-}Rb_1$,
- 10 to 60 % by weight of saponin $G\text{-}Rg_1$,

- 0 to 15 % by weight of saponin G-Rd,
- 0 to 15 % by weight of saponin $N-R_1$,
- 1 to 10 % by weight of saponin G-Re,

with respect to the total weight of the extract.

**8.** Use according to one of claims 5 to 7, **characterised in that** said ginsenoside $G-Rb_1$ is the saponin mainly contained in said extract.

**9.** Use according to one of claims 5 to 8, **characterised in that** said plant of the Panax type is Panax notoginseng or Panax quinquefolium.

**10.** Use according to one of claims 5 to 9, **characterised in that** said extract is an extract of Panax notoginseng, preferably of subterranean parts of the plant.

**11.** Use according to one of claims 5 to 9, **characterised in that** said extract is an extract of Panax quinquefolium, preferably an extract of a subterranean part of this plant.

**12.** Use according to one of claims 5 to 11, **characterised in that** said extract is obtained by extraction by means of a polar solvent or of a mixture of polar solvents.

**13.** Use according to one of claims 5 to 12, **characterised in that** said extract is obtained by extraction by means of a solvent or of a mixture of solvents selected from the group constituted by water, alcohols comprising preferably 1 to 4 carbon atoms, particularly methanol, ethanol, propanol, isopropanol, propyleneglycol and butyleneglycol.

**14.** Use according to claim 13, **characterised in that** the solvent or mixture of solvents is selected from the group constituted by methanol, ethanol, 1,3-butyleneglycol and mixtures of these solvents by themselves or with water.

**15.** Use according to one of claims 1 to 14, **characterised in that** said composition contains, further, ascorbic acid, particularly L-ascorbic acid or vitamin C or its isomer erythorbic acid or one of their salts or esters, designated under the generic name of ascorbic derivatives.

**16.** Use according to one of claims 1 to 14, **characterised in that** said composition contains, further, an effective amount of an active principle stimulating the synthesis of the components of the extracellular matrix of the dermis, particularly of collagen, particularly of collagen I and of collagen III, and of glycosaminoglycans.

**17.** Use according to claim 16, **characterised in that** said composition contains an active principle stimulating the synthesis of collagen, particularly of collagen I and of collagen III, selected from the group constituted by madecassoside, an extract of centella asiatica and an extract of ginseng, particularly ginsenoside Ro, at a concentration by weight of 0.01 % to 5 %.

**18.** Use according to claim 16, **characterised in that** the composition contains an active principle stimulating the synthesis of glycosaminoglycans, selected from the group constituted by an extract of Filicium decipiens, particularly an extract of the bark of the root of this plant, an extract of Eriobotrya japonica and PDGF.

**19.** Use according to one of claims 1 to 18, **characterised in that** said composition contains, further, vitamins, particularly vitamin A, its palmitic, propionic or acetic ester or vitamin E and its derivatives, at a concentration by weight of 0.0001 to 5 % with respect to the total weight of said composition.

**20.** Method of cosmetic treatment intended to prevent or to correct a loss of skin elasticity, tonicity or firmness, **characterised in that** it comprises the application on the areas of the skin to be treated an effective amount of ginsenoside $Rb_1$ or of a plant extract containing same to stimulate the synthesis of elastin by the fibroblasts of the dermis, said ginsenoside being incorporated in a cosmetically acceptable excipient.